# FASCICULE DE BREVET EUROPEEN

(11) **EP 2 051 797 B1**
(45) Date de publication et mention de la délivrance du brevet: **30.06.2010**
(21) Numéro de dépôt: 07823266.7
(22) Date de dépôt: 06.07.2007
(51) Int. Cl.: B01D 53/22, B01D 71/64, B01D 69/08, B01D 71/32, B01D 71/56, C07C 7/144, C07C 9/10, C07C 11/08, C10G 31/11

(54) **PROCEDE DE SEPARATION PAR MEMBRANE D'HYDROCARBURES LINEAIRES AU SEIN D'UN MELANGE D'HYDROCARBURES**
VERFAHREN ZUR MEMBRANTRENNUNG LINEARER KOHLENWASSERSTOFFE IN EINER MISCHUNG VON KOHLENWASSERSTOFFEN
PROCESS FOR MEMBRANE SEPARATION OF LINEAR HYDROCARBONS WITHIN A MIXTURE OF HYDROCARBONS

(30) Priorité: 09.08.2006 FR 0607277
(43) Date de publication de la demande: 29.04.2009
(73) Titulaire: IFP, 92852 Rueil-Malmaison Cédex (FR)
(72) Inventeur: GONZALEZ, Serge, 69150 Décines (FR); VALLET, Jacques, 69008 Lyon (FR); RODESCHINI, Hélène, 69003 Lyon (FR); BAUDOT, Arnaud, 69390 Vernaison (FR)
(86) Numéro de dépôt international: PCT/FR2007/001173
(87) Numéro de publication internationale: WO 2008/017744

(56) Documents cités:
- EP-A- 0 412 882
- EP-A- 0 545 686
- US-A1- 5 409 525
- US-B1- 6 531 569
- US-B2- 6 899 743
- ISLAM ET AL: "Preparation and gas separation performance of flexible pyrolytic membranes by low-temperature pyrolysis of sulfonated polyimides" JOURNAL OF MEMBRANE SCIENCE, ELSEVIER SCIENTIFIC PUBL.COMPANY. AMSTERDAM, NL, vol. 261, no. 1-2, 15 septembre 2005 (2005-09-15), pages 17-26, XP005043832 ISSN: 0376-7388

## Description

### DOMAINE DE L'INVENTION

L'invention concerne un procédé permettant de séparer des hydrocarbures linéaires d'hydrocarbures branchés au moyen d'une membrane comprenant une couche sélective dense constitué d'un polymère dont la structure chimique contient un groupement bis-phényl-9,9-fluorène.
La présente invention convient particulièrement à la séparation des isomères linéaires et des isomères branchés.
La présente invention s'applique également à la séparation d'isomères de type paraffines ou de type oléfines.
La présente invention trouve une application particulièrement intéressante dans la séparation d'une fraction de normales paraffines contenue dans une coupe hydrocarbonée à nombre d'atomes de carbone allant de 4 à 16, et plus particulièrement à nombre d'atomes de carbone allant de 4 à 10, et par exemple, pour un nombre d'atomes de carbone égal à 4, 5 ou 6.

### EXAMEN DE L'ART ANTERIEUR

Dans les documents de l'art antérieur, les performances de séparation des membranes sont généralement décrites au moyen de deux paramètres: la perméabilité et la sélectivité.
La perméabilité est définie comme la densité de flux de matière traversant la membrane, rapportée à l'épaisseur de ladite membrane, et à la différence de pression partielle des composés traversant la membrane appliquée entre les faces amont et aval.
La sélectivité de la membrane pour le constituant A par rapport au constituant B est définie comme le rapport des perméabilités des deux constituants A sur B.
La perméabilité est mesurée en barrer ( 1 barrer = 10⁻¹⁰ cm³.cm/cm².cm_{Hg}, soit en unité SI 0,75 10⁻¹⁵ Nm³.m/m².s.Pa).
Dans le cas de la séparation d'un mélange binaire, le facteur de séparation peut être calculé de deux manières; soit à partir des perméabilités obtenues en corps pur (on parle alors de sélectivité idéale ou de permsélectivité), soit à partir des données des flux en mélange (on parle alors de sélectivité en mélange ou de facteur de séparation).

Le procédé de séparation décrit dans la présente invention est réalisé par un mécanisme de solution/diffusion au travers d'un film polymère dense formant la couche sélective de la membrane.
De manière générale, les membranes offrant une grande sélectivité sont très peu perméables, et inversement, une membrane très perméable présente généralement des valeurs de sélectivité assez faibles.
La séparation de molécules de point d'ébullition proche, ou de nombre d'atomes de carbone voisins, est très employée en raffinerie et s'applique à diverses coupes pétrolières.
Les molécules concernées sont le plus souvent les paraffines de différents degrés de branchement et dans une moindre mesure, les composés oléfiniques. Plus généralement la présente invention s'applique à la séparation d'isomères, quelle que soit la famille chimique à laquelle appartiennent ces isomères. Le plus souvent, il s'agira d'isomères paraffiniques, ou d'isomères oléfiniques.
Ces séparations sont généralement effectuées par distillation, par adsorption ou selon la technique dite du contre courant simulé et présentent toutes des inconvénients bien connus en terme de coûts énergétiques ou de facilité d'opération.
La technique de séparation par membrane est beaucoup moins courante en raffinage, mais présente des avantages certains, en termes de modularité, consommation énergétique faible comparée à une distillation conventionnelle, coûts de maintenance réduits du fait de l'absence d'éléments mobiles, et aptitude à effectuer des séparations difficiles.

Les membranes minérales à base de tamis moléculaires sont les plus indiquées pour réaliser la séparation de mélanges d'isomères.
Ainsi le brevet US 5914434 présente une membrane en carbone pour séparer les alcanes linéaires des alcanes branchés selon un mécanisme de sélectivité diffusionnelle.
Les membranes zéolithiques de type MFI sont les plus couramment citées dans la littérature. Ces membranes zéolithiques sont choisies principalement parce que le diamètre des micropores (de l'ordre de 5,5 angströms) est supérieur au rayon cinétique minimum des paraffines linéaires (et à fortiori des oléfines linéaires) ce qui permet une diffusion rapide de ce type de molécules, tout en étant inférieur à celui des isomères paraffiniques ou oléfiniques monobranchés, et a fortiori multibranchés, qui diffuseront nettement plus lentement que leurs homologues linéaires.

De plus, les zéolithes sont des tamis qui offrent une résistance accrue aux températures élevées et en présence de composés organiques, ce qui permet d'utiliser ce type de membranes en couplage avec des réacteurs haute température utilisés dans l'industrie du raffinage.
La plupart des travaux de R&D dans le domaine de la synthèse des membranes zéolithes ont visé à produire une couche de zéolithe intègre la plus fine possible. A notre connaissance, la membrane zéolithe offrant la couche sélective la plus fine (0,5 µm d'épaisseur) ayant fait l'objet d'une publication est décrite dans un article de Hedlund *et al*. (Jonas Hedlund, Johan Sterte, Marc Anthonis, Anton-Jan Bons,Barbara Carstensen Ned Corcoran, Don Cox, Harry Deckman,Wim De Gijnst, Peter-Paul de Moor, Frank Lai, Jim McHenry,Wilfried Mortier, Juan Reinoso, Jack Peters, Microporous and Mesoporous Materials 52 (2002) 179-189). Le titres de la revue citée peut être traduit en français par " matériaux microporeux et mésoporeux".
Du fait du comportement solide de ce type de film zéolithe, il est obligatoire d'effectuer la synthèse de la couche sélective zéolithe sur un support poreux, le plus souvent métallique ou à base d'oxydes (généralement de l'alumine).
Malgré les avantages offerts théoriquement par les membranes zéolithes, une analyse approfondie de la littérature scientifique montre que ce type de matériaux présente des inconvénients importants:
(1) la difficulté d'obtenir ou de maintenir une couche sélective sans défauts intercristallins lors des essais de séparation en température
(2) leur réactivité notamment en présence de composés réactifs tels que les oléfines
(3) la difficulté de synthétiser des couches minces à grande échelle, du fait de la nature "discrète" des cristaux de zéolithes constitutifs de la couche sélective
(4) le coût élevé des supports métalliques ou minéraux

Concernant le premier inconvénient, de nombreux auteurs, par exemple Stuart M. Holmes, Christian Markert, Richard J. Plaisted, James O. Forrest, Jonathon R. Agger, Michael W. Anderson, Colin S. Cundy, and John Dwyer, Chem. Mater. 1999, 11, 3329-3332 ( traduction du titre de la revue citée "Matériaux chimiques") ont montré que des fissures intercristallines apparaissaient lors de la calcination de la couche zéolithique après synthèse ou du fait de la différence de dilatation entre le support poreux et la couche sélective en zéolithe.

Ces défauts, et particulièrement lorsque les températures sont élevées, peuvent altérer fortement la sélectivité des membranes zéolithes. De nombreux auteurs dont Vu Anh Tuan, John L. Falconer, and Richard D. Noble, Ind. Eng. Chem. Res. 1999, 38, 3635-3646 ( traduction du titre de la revue citée "Recherche en génie chimique") ont ainsi pu observer que si les membranes zéolithes de type MFI offraient généralement aux basses températures des sélectivités en mélanges élevées pour la séparation du mélange normal butane/isobutane, la sélectivité pouvaient diminuer fortement avec la température. Ainsi les sélectivités de membranes zéolithes de type MFI à une température supérieure à 100°C sont généralement assez réduites, comme décrit dans le tableau n°1.

**Tableau n°1. Données publiées dans la littérature de sélectivités normal butane / isobutane de membranes zéolithes de structure ZSM-5 à des températures élevées.**

| Nature couche sélective | Température test de séparation | sélectivité en mélange | référence |
|---|---|---|---|
| ZSM-5 | 200°C | 4,8 | Tuan *et al*., 1999 |
| ZSM-5 | 200°C | 5,2 | Tuan *et al*., 1999 |
| ZSM-5 | 130°C | 2,8 | Bernal *et al*., 2002 |
| ZSM-5 | 130°C | 3 | Bernal *et al*., 2002 |
| ZSM-5 | 130°C | 13,4 | Bernal *et al*., 2002 |
| ZSM-5 | 150°C | 2,5 à 4 | Jareman *et al*., 2003 |
| ZSM-5 | 150°C | 5 | Nishiyama *et al*., 2001 |
| Na-ZSM-5 | 200°C | 2 | Aoki *et al*., 2000 |
| H-ZSM-5 | 200°C | 1,5 | Aoki *et al*., 2000 |
| Ca-ZSM-5 | 200°C | 2 | Aoki *et al*., 2000 |
| K-ZSM-5 | 200°C | 2 | Aoki *et al*., 2000 |
| Ba-ZSM-5 | 200°C | 2,5 | Aoki *et al*., 2000 |
| ZSM-5 | 160°C | 3 | Hedlund *et al*., 2002 |
| ZSM-5 | 150°C | 10 | Coronas *et al*., 1998 |

Le deuxième inconvénient des membranes zéolithes concerne leur réactivité, notamment en présence de composés réactifs tels que les oléfines. En effet, il est connu de l'homme du métier que la plupart des zéolithes contiennent de l'aluminium qui confère un caractère acide à la microporosité de la zéolithe.
La réactivité des atomes d'aluminium dans le réseau de la zéolithe conduit généralement in *fine* à un bouchage des pores de la zéolithe par oligomérisation des oléfines en leur sein.
Ce phénomène est d'autant plus marqué que le mélange à séparer contient des composés de type isobutène.
Plus généralement, la difficulté de synthétiser des couches minces à grande échelle, du fait de la nature "discrète" des cristaux de zéolithes constitutifs de la couche sélective, associée au coût élevé des supports métalliques ou minéraux poreux nécessaires au maintien de la couche sélective des membranes à base de tamis moléculaire, ont fait que ce type de membranes n'a pas connu le développement industriel escompté. En fait, une seule entreprise, Mitsui, continue la production d'unités de séparation à base de membranes zéolithes de petite taille (surface inférieure à 100 m²) alors qu'une autre, la société suisse Sulzer, a abandonné la fabrication de membrane à base de silice mésoporeuse du fait de leur mauvaise tenue en température.
A l'inverse des zéolithes, les polymères présentent de nombreux atouts, notamment en terme de mise en forme. En effet, de nombreux polymères peuvent être mis sous la forme de film denses très minces. Cette capacité a été mise à profit à partir des années 70 pour produire des membranes de séparation de gaz permanents et des sociétés se sont faits une spécialité de produire des membranes de séparation de gaz sous la forme de fibres creuses de diamètre très faible, ce qui permettait d'offrir des équipements de séparation membranaire extrêmement compacts.
Les applications courantes pour ce type de membranes sont :
- la séparation azote/oxygène de l'air
- la purification de l'hydrogène
- la séparation CO₂/CH₄ dans l'industrie du gaz naturel

Très peu de documents de l'art antérieur font référence à l'usage de membranes polymères pour la séparation de mélange d'hydrocarbures. En effet, il est généralement admis par l'homme de l'art que les hydrocarbures provoquent des phénomènes de gonflement des matrices polymères qui perdent alors leurs propriétés de tamisage, ce qui se traduit généralement par des sélectivités très faibles. Du coup, les éléments de l'art antérieur faisant référence à la séparation de mélange de composés organiques comportant au moins 4 atomes de carbone sont très peu nombreux.
On peut citer le brevet US 6899743 dans lequel il est montré qu'il est possible de séparer un mélange de normal butane et d'isobutane à travers un film dense en Hyflon AD60x, polymère fluoré produit par la société Solvay. Le film présente une perméabilité vis-à-vis du normal butane plus élevée que pour l'isobutane. Toutefois, il apparaît clairement que la sélectivité de la séparation décroît très fortement avec la température.

**Tableau 2. Données de sélectivité normal butane / isobutane sur membranes denses à base de Hyflon AD 60x (brevet US 6899743).**

| température | sélectivité n-butane/iso-butane |
|---|---|
| 20°C | 8,9 |
| 40°C | 6,9 |
| 60°C | 5,6 |
| 80°C | 4,3 |

EP-A-0545686 décrit un procédé de séparation des hydrocarbures linéaires et branchés au moyen d'une membrane constituée d'un polymère contenir un diamide aromatique.

Islam et al (J Mem Sci 261, 2005, p.17-26) décrit une membrane contenir un groupement 9,9-bis (4-amino phényl) fluorène pour la séparation des oléfines et des paraffines.

Dans le cadre de la présente invention il a été découvert de manière surprenante que les polymères dont le motif moléculaire comprenait un groupement bis-phényl-9,9-fluorène permettaient de séparer très efficacement les paraffines ou les oléfines linéaires des paraffines ou oléfines branchées. Les sélectivités composés linéaires / composés branchés observées avec les films à base des polymères décrits dans la présente invention sont plus élevées que celles publiées dans la littérature, de même que leur perméabilité vis-à-vis de composés linéaires.

### DESCRIPTION SOMMAIRE DE L'INVENTION

La présente invention appartient au domaine des procédés de séparation par membrane et s'applique à la séparation d'hydrocarbures linéaires contenus dans un mélange d'hydrocarbures linéaires et d'hydrocarbures branchés.
Par exemple, le présent procédé permet de séparer le normal butane d'un mélange contenant d'autres hydrocarbures en C4 tels que l'isobutane.
Par exemple, le présent procédé permet de séparer les normal butènes d'un mélange contenant d'autres hydrocarbures en C4 tels que les isobutènes.
Il est montré dans la présente invention que la présence d'un groupement particulier de type bis-phényl-9,9-fluorène dans un polymère rigide mis sous forme de film dense conduit à des propriétés supérieures de séparation, notamment en terme de perméabilité dudit film vis-à-vis des hydrocarbures linéaires, tout en maintenant une sélectivité composé linéaire/composé branché élevée.
Plus généralement le procédé selon la présente invention s'applique à la séparation d'une fraction de normales paraffines ou de normales oléfines, contenue dans une coupe hydrocarbonée à nombre d'atomes de carbone allant de 4 à 16, et plus particulièrement à nombre d'atomes de carbone allant de 4 à 10. De manière particulièrement préférée, le procédé selon l'invention s'applique à la séparation de normales paraffines ou de normales oléfines à 4, 5 ou 6 atomes de carbone. Dans la suite du texte on regroupera les différents cas de figure en parlant de séparation d'hydrocarbures linéaires à 4, 5 ou 6 atomes de carbone.
Les membranes utilisées dans le procédé selon la présente invention sont des membranes de type polymère amorphe vitreux ou présentant une faible cristallinité comprenant, dans le motif de répétition, au moins un groupement bis-phényl-9,9-fluorène.
L'invention consiste donc en un procédé de séparation par membrane dans lequel la couche sélective de la membrane polymère est constituée d'un film dense polymère dont la structure chimique contient un groupement-bis-phényl-9,9-fluorène.
La couche sélective de la membrane polymère contenant le groupement bis-phényl-9,9-fluorène est sélectionnée dans le groupe constitué par les polymères des familles suivantes: les polyimides, les polyamides, les polycarbonates, les polysulfones, les poly(amides imides), les poly(éther sulfones), les polyesters, ou par les copolymères ou mélanges de polymères de ces familles.
De façon préférée, la couche sélective de la membrane polymère contenant le groupement bis-phényl-9,9-fluorène appartient à la famille des polyamides.
De façon également préférée, la couche sélective de la membrane polymère contenant le groupement bis-phényl-9,9-fluorène appartient à la famille des polycarbonates.
De façon encore davantage préférée, la couche sélective de la membrane polymère contenant le groupement bis-phényl-9,9-fluorène appartient à la famille des polyimides.

### DESCRIPTION DETAILLEE DE L'INVENTION

La présente invention appartient au domaine des procédés de séparation par membrane et s'applique à la séparation d'hydrocarbures linéaires contenus dans un mélange d'hydrocarbures linéaires et d'hydrocarbures branchés.
Par exemple, le présent procédé permet de séparer le normal butane d'un mélange contenant d'autres hydrocarbures en C4 tels que l'isobutane.
Par exemple, le présent procédé permet de séparer les normal butènes d'un mélange contenant d'autres hydrocarbures en C4 tels que les isobutènes.
Il est montré dans la présente invention que la présence d'un groupement particulier de type bis-phényl-9,9-fluorène dans un polymère rigide mis sous forme de film dense conduit à des propriétés supérieures de séparation, notamment en terme de perméabilité dudit film vis-à-vis des hydrocarbures linéaires, tout en maintenant une sélectivité composé linéaire/composé branché élevée.
Plus généralement Le procédé selon la présente invention s'applique à la séparation d'une fraction de normales paraffines ou de normales oléfines contenue dans une coupe hydrocarbonée à nombre d'atomes de carbone allant de 4 à 16, et plus particulièrement à nombre d'atomes de carbone allant de 4 à 10. De manière particulièrement préférée, le procédé selon l'invention s'applique à la séparation d'hydrocarbures linéaires à 4, 5 ou 6 atomes de carbone.
Les membranes utilisées dans le procédé selon la présente invention sont des membranes de type polymère amorphe, vitreux ou présentant une faible cristallinité, comprenant, dans le motif de répétition, au moins un groupement bis-phényl-9,9-fluorène.

L'invention consiste donc en un procédé de séparation par membrane dans lequel la couche sélective de la membrane polymère est constituée d'un film dense polymère dont la structure chimique contient un groupement bis-phényl-9,9-fluorène.
La couche sélective de la membrane polymère contenant le groupement bis-phényl-9,9-fluorène est sélectionnée dans le groupe constitué par les polymères des familles suivantes: les polyimides, les polyamides, les polycarbonates, les polysulfones, les poly(amides imides), les poly(éther sulfones), les polyesters, ou par les copolymères ou mélanges de polymères de ces familles.
- Dans une première variante de l'invention, la couche sélective de la membrane polymère contenant le groupement bis-phényl-9,9-fluorène appartient à la famille des polyimides.
- Dans une seconde variante de l'invention, la couche sélective de la membrane polymère contenant le groupement bis-phényl-9,9-fluorène appartient à la famille des polyamides.
- Dans une troisième variante de l'invention, la couche sélective de la membrane polymère contenant le groupement bis-phényl-9,9-fluorène appartient à la famille des polycarbonates.
- Dans une quatrième variante de l'invention, la couche sélective de la membrane polymère contenant le groupement bis-phényl-9,9-fluorène appartient à la famille des polysulfones.
- Dans une cinquième variante de l'invention, la couche sélective de la membrane polymère contenant le groupement bis-phényl-9,9-fluorène appartient à la famille des poly(amides imides).
- Dans une sixième variante de l'invention, la couche sélective de la membrane polymère contenant le groupement bis-phényl-9,9-fluorène appartient à la famille des poly(éther sulfones).
- Dans une septième variante de l'invention, la couche sélective de la membrane polymère contenant le groupement bis-phényl-9,9-fluorène appartient à la famille des polyesters.
De façon très préférée, la couche sélective de la membrane polymère contenant le groupement bis-phényl-9,9-fluorène appartient à la famille des polyimides.

Le polymère constitutif de la couche sélective de la membrane peut être un homopolymère, un copolymère, un mélange de polymères.
Les membranes utilisées dans la présente invention, outre la présence du polymère vitreux comprenant dans le motif de répétition au moins un groupement bis-phényl-9,9-fluorène, pourront contenir des charges minérales et organiques et additifs destinés à entraîner de part leur présence une amélioration du facteur de séparation et/ou favoriser la perméabilité. On pourra à titre d'exemple citer les charges minérales telles que les sels métalliques, les zéolites, les argiles, les composés mésoporeux, les silices natives ou post-traitées, les noirs de carbone, les polymères pyrolysés, les nanotubes de carbones, les dendrimères.
Les membranes utilisées dans la présente invention, outre la présence du polymère vitreux comprenant dans le motif de répétition au moins un groupement bis-phényl-9,9-fluorène, pourront contenir des agents de réticulation entraînant une amélioration du facteur de séparation et/ou favoriser la perméabilité.
Les membranes utilisées dans la présente invention pourront être traitées chimiquement, thermiquement, ou par rayonnement en vue d'améliorer le facteur de séparation et/ou de favoriser la perméabilité.
Le polymère constitutif de la couche sélective de la membrane selon l'invention comporte dans le motif de répétition au moins un groupement bis-phényl-9,9-fluorène de formule chimique générale : 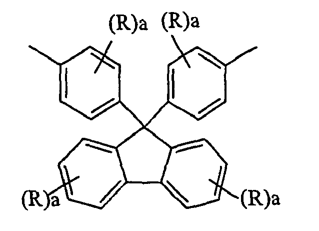 dans lequel, chacun des groupes R représente soit un groupe alkyle, linéaire ou ramifié ayant de 1 à 16 atomes de carbone, soit un groupe alkoxy, linéaire ou ramifié ayant de 1 à 16 atomes de carbone. L'indice a peut prendre pour chacun des groupes R et de manière indépendante d'un groupe à l'autre, soit la valeur zéro, soit une valeur entière comprise entre 1 et 4. Préférentiellement chaque valeur de l'indice a sera 0 ou 1.
De manière encore plus préférée, l'indice a aura une valeur nulle, ce qui revient à la suppression des groupes R.
Pour les groupes alkyle, on peut citer de manière non limitative les groupes méthyle, éthyle, propyle, isopropyle, les groupes butyle linéaires ou ramifiés.
Pour les groupes alkoxy, on peut citer de manière non limitative les groupes méthoxy, éthoxy, propyloxy, et les groupes butyloxy linéaires ou ramifiés.

Dans une version préférée, le polymère constitutif de la couche sélective de la membrane sera un homopolymère, ou un copolymère de formule générale : 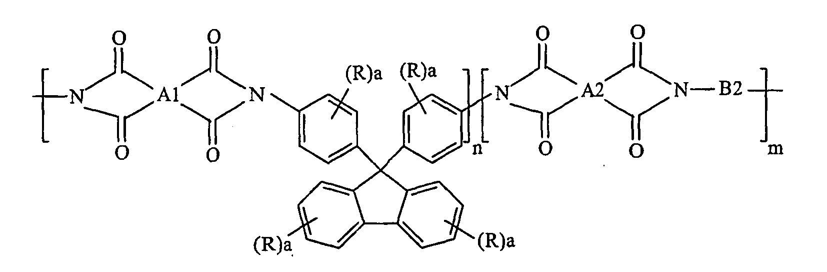 où A1 et A2 sont des groupements organiques tétravalents hydrocarbonés choisis parmi des groupes d'hydrocarbures aromatiques, alicycliques et aliphatiques, et le groupe B2 est un groupement organique bivalent hydrocarboné choisi parmi des groupes d'hydrocarbures aromatiques, alicycliques et aliphatiques. Les indices m et n représentent un nombre entier positif qui correspond au degré de polymérisation.
Dans une version préférée, le polyimide constitutif de la couche sélective de la membrane est un polymère statistique, alterné, séquencé ou block.

La voie la plus généralement utilisée pour l'obtention du polyimide constitutif de la couche sélective de la membrane résulte de la réaction chimique entre
- une diamine comportant dans sa structure le groupement bis-phényl-9,9-fluorène de formule générale :

   H₂N-B2-NH₂
- et un dianhydride de formule générale :

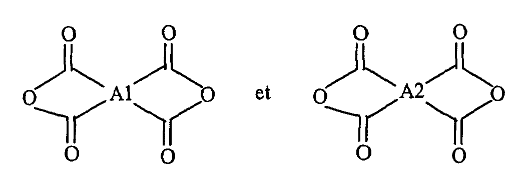

On pourra dans le cadre de l'invention utiliser un précurseur tel que par exemple un tétra acide carboxylique, ou l'hémiester d'un tétra acide carboxylique.

Dans le cadre de l'invention la diamine peut être choisie dans la liste suivante:
- 1,4-diamino-2,3,5,6-tétraméthylbenzène
- bis(4-aminophényl) éther
- 2,4-diamino-1-isopropylbenzène
- les diaminoanthraquinones
- 2,7-diaminofluorène
- 4,4'-diamino-3,3'-diméthoxybiphényl
- 2,4-diaminotoluène
- diaminodiphénylsulfone
- bis[4-(4-aminophénoxy)phényl]sulfone
- 9,10-bis(4-aminophényl)anthracène
- 1,4-bis(4-aminophényl)benzène
- bis(4- aminophényl)méthane
- bis(4-amino-3-éthylphényl)méthane
- bis(4-amino-3-méthylphényl)méthane
- bis(4-amino-3-chloro-phényl)méthane
- bis(4-aminophényl)sulfide
- 2,2-bis(4-amino-3-hydroxyphényl)propane
- 4,4'-diamino-3,3'-dichlorobiphényl
- 4,4'-diamino- 3,3'-dihydroxybiphényl
- 4,4'-diaminobiphényl
- 9,9-bis(4-aminophényl)fluorène
- bis(4-amino-2,6-méthylphényl) méthane
- 1,4-diamino-2,5-dichlorobenzène
- 1,4-dianùno-2,5-diméthylbenzène
- 1,3-diamino-2,4,6-triméthylbenzène
- bis(3-aminopropyl)tétraméthyldisiloxane
- 2,5-diaminopyridine
- 4,4'-diaminobenzanilide
- 1,5-diaminonaphthalène
- 1,3- diamino-5-trifluorométhylbenzène
- 4,4'-diamino-3,3',5,5'-tétraméthylbiphényl
- 3,3'-diamino-4,4'-dihydroxybiphényl
- 1,3-phénylènediamine
- 1,4-phénylènediamine
- 1,4-bis(4-aminophenoxy)benzène.

De manière préférée on choisira les diamines dans la liste suivante:
- la 1,4-diamino-2,3,5,6-tétraméthylbenzène
- 9,9-bis(4-aminophényl)fluorène
- 1,3-diamino-2,4,6-triméthylbenzène
- bis(3-aminopropyl)tétraméthyldisiloxane

Dans le cadre de l'invention, le dianhydride choisi peut être choisie dans la liste suivante:
- le dianhydride de l'acide bis(3,4-dicarboxyphényl)sulfone
- le dianhydride de l'acide 2,2-bis(3,4-dicarboxyphényl)hexafluoropropane
- le dianhydride de l'acide 1,1-bis(3,4-dicarboxyphényl)éthane
- l'anhydride pyromellitique
- le dianhydride de l'acide 2,3,6,7-naphthalenetétracaxboxylique
- le dianhydride de l'acide 3,3',4,4'-biphényltétracarboxylique
- le dianhydride de l'acide 1,2,5,6-naphthalenetétracarboxylique
- le dianhydride de l'acide 2,2'3,3'-biphényltétracarboxylique
- le dianhydride de l'acide 3,3',4,4'-benzophenonetétracarboxylique
- le dianhydride de l'acide oxydiphthalique
- le dianhydride de l'acide 1,4,5,8-naphthalènetéiracarboxylique
- le dianhydride de l'acide 2,2-bis(3,4-dicarboxyphényl)propane
- le dianhydride de l'acide 3,4,9,10-pérylènetétracarboxylique
- le dianhydride de l'acide 1,1-bis(2,3-dicarboxyphényl)éthane
- le dianhydride de l'acide bis(2,3-dicarboxyphényl)méthane
- le dianhydride de l'acide bis(3,4-dicarboxyphényl)méthane.

On choisira de manière préférée le dianhydride dans la liste suivante:
- le dianhydride de l'acide 2,2-bis(3,4-dicarboxyphényl)hexafluoropropane
- l'anhydride pyromellitique
- le dianhydride de l'acide 3,3',4,4'-biphényltétracarboxylique
- le dianhydride de l'acide 3,3',4,4'-benzophenonetétracarboxylique

Les solvants nécessaires à la réalisation de la polymérisation peuvent être choisi dans la liste suivante:
- N,N-diméthylformamide
- N,N-diéthylformamide
- N,N- diméthylacétamide (DMAC)
- N,N-diéthylacétamide
- N-méthyl-2-pyrrolidone (NMP)
- N-cyclohexyl-2-pyrrolidone
- phénol
- o-, m-, p-crésol
- xylénol
- phénols hallogénés
- catéchol
- hexaméthylphosphoramide
- diméthylpropyl urée
- alcools benzyliques
- lactates
- lactones telles que la γ- butyrolactone.

Les solvants seront choisis de manière préférée dans la liste suivante:
- N,N- diméthylacétamide (DMAC)
- N-méthyl-2-pyrrolidone (NMP)
- o-, m-, p-crésol
- lactones telles que la γ- butyrolactone.

Ces solvants peuvent être utilisés seuls ou en mélange.

La connaissance de la masse moléculaire du polymère n'est pas indispensable, et l'on préférera suivre l'évolution de la viscosité inhérente du polymère qui doit être au moins supérieure à 0,1 dl/g et préférablement comprise entre 0,3 dl/g et 2 dl/g. La viscosité inhérente se définit par rapport à une viscosité de référence et à la concentration du polymère en solution dans le solvant. Sa valeur est homogène à l'inverse de ladite concentration soit 1dl/g (=0,1 m³/kg).
La plupart des polymères envisagés dans la présente invention pour une mise en oeuvre sous forme de membrane sont solubles dans une grande variété de solvants organiques communs incluant la plupart des solvants aprotiques, qui sont généralement utilisés pour la formation de membranes polymères comme la NMP.
La membrane polymère contenant le groupement bis-phényl-9,9-fluorène peut être homogène ou asymétrique.
Le polymère constitutif de la couche sélective de la membrane peut être mis en oeuvre sous forme de film ou de fibres selon les techniques connues de l'homme du métier.
Une fois synthétisé, le polymère sous la forme d'un solide est dissout dans un solvant approprié comme la NMP par exemple, à une teneur en polymère de l'ordre de 1% à 50% en masse, et préférablement comprise entre 5% et 20% en masse.
La solution est étendue sous forme de film à l'épaisseur désirée sur un support plan ou sur un support se présentant sous forme de fibres creuses, ou bien est extrudée au travers d'une fileuse conventionnelle.

Il est possible de réaliser la membrane, et l'on parlera alors de membrane composite, en déposant un film de polymère comportant dans sa chaîne au moins un groupement bis-phényl-9,9-fluorène d'une épaisseur comprise entre 0,05 et 1 micron (1 µm = 10⁻⁶ mètre) sur un support préalablement mis en oeuvre sous forme de fibre creuse.
On choisira avantageusement le support de telle manière qu'il présente l'avantage d'être beaucoup plus perméable que les polyimides en général, et ne contribue pas significativement à la résistance au transfert de matière à travers la membrane composite résultante.
Selon un mode de réalisation de l'invention, le support sera une couche poreuse ou une fibre creuse constituée d'un matériau polymère tel que par exemple un polysulfone, un polyéthersulfone, un polyétherimide, un polyfluorure de vinylidène, un poléthylène ou un polypropylène, un polyacrylonitrile, un polyimide, un polyoxyde de phénylène, un polymère dérivé de la cellulose tel qu'un acétate de cellulose ou un éthylcellulose. Le support pourra être un polymère constitué de différents matériaux organiques ou minéraux.
L'adhésion entre la couche sélective et le support nécessite dans certains cas des traitements physiques ou chimiques qui sont bien connus de l'homme de l'art.

Dans la suite du texte, on prendra comme exemple la séparation du normal butane contenu dans un mélange de normal et d'iso butane. Mais il convient de garder à l'esprit que le présent procédé s'applique aussi bien à la séparation du butène-1 d'un mélange contenant d'autres hydrocarbures comme l'isobutène.

Les membranes de la présente invention peuvent être utilisées dans divers types de modules destinés à la réalisation de l'unité de séparation. Le module de séparation final peut être constitué d'une ou de plusieurs membranes. Le module peut être assemblé à d'autres modules identiques de manière à former une unité de séparation ayant la taille désirée.
En fonctionnement, la charge constituée d'un mélange d'hydrocarbures linéaires et branchés est mise en contact avec un des cotés de la membrane.
Par charge hydrocarbure, on entend préférentiellement dans le cadre de la présente invention les paraffines ou les oléfines comprenant au moins 4 atomes de carbone.
En imposant une différence de pression entre le coté de la charge et le coté perméat, les composés linéaires traversent la membrane à une vitesse plus importante que les composés branchés comprenant le même nombre d'atomes de carbone. Cette différence de vitesse produit un flux hydrocarboné enrichi en composés linéaires, qui est prélevé du coté perméat de la membrane.
La présente invention n'est pas destinée uniquement aux séparations en phase gaz mais peut être étendue à d'autres types de séparation, en phase liquide par exemple, et ceci pour des conditions de température et de pression couvrant un large domaine d'utilisation. En outre, la séparation peut avoir lieu pour des mélanges contenant plus de deux composants.
De manière large, le procédé de séparation par membrane selon l'invention fonctionne à une température comprise entre 40 et 250°C, pour des pressions comprises entre 0,1 MPa et 5 MPa (1 bar= 0,1 MPa).
De manière préférée, le procédé de séparation par membrane selon l'invention fonctionne à une température comprise entre 40 et 200°C, pour des pressions comprises entre 1 bar et 40 bar. De manière encore davantage préférée, le procédé de séparation par membrane selon l'invention fonctionne à une température comprise entre 50 et 150°C, pour des pressions comprises entre 0,1 MPa et 2 MPa.

### EXEMPLES SELON L'INVENTION

### exemple 1 ( selon l'invention)

Le polymère 6FDA-BDAF faisant l'objet de l'exemple 1 est le résultat de la polycondensation du dianhydride de l'acide 2,2-bis(3,4-dicarboxyphényl)hexafluoropropane (6FDA) et de la 9,9'bis-(4-aminophényl)-fluorène (BDAF) en mélange équimolaire. Après purification des monomères par recristallisation dans des solvants appropriés, la polycondensation du polyimide est réalisée en deux étapes: dans un premier temps, le polyamide acide est réalisé, puis le polyimide est obtenu dans une deuxième étape de cyclisation par voie chimique.
Lors de la première étape de polymérisation, le mélange de la dianhydride et de la diamine est effectué sous atmosphère inerte et en milieu anhydre dans le solvant N-N-diméthylacétamide (DMAC).
L'étape de cyclo-déshydratation est effectuée par ajout goutte à goutte d'un mélange cyclisant composé de triéthylamine et d'anhydride acétique en mélange dans le solvant de synthèse.
Le polyimide ainsi obtenu est alors précipité dans de l'eau puis broyé. Il est ensuite filtré, rincé, puis séché à l'étuve sous vide en augmentant progressivement la température jusqu'à atteindre 150°C.
La viscosité inhérente du polymère ainsi obtenu est de 1,3 dl/g.
Le matériau sous forme de broyé est ensuite mis en solution dans de la DMAC à une concentration massique de 12% sous l'effet d'une bonne agitation mécanique à température ambiante.
La solution limpide est ensuite filtrée sous une pression de 0,2 MPa sur un filtre de type Millipore ayant un seuil de coupure de 1 µm.
Cette solution est ensuite mise sous forme d'un film à l'aide d'une barre spiralée de 300 µm sur une plaque de verre préalablement dégraissée à l'acétone, puis séchée.
La plaque est introduite dans une étuve. L'évaporation du solvant est effectuée par une élévation progressive de la température jusqu'à 200°C. La température finale est maintenue en palier pendant deux heures. Après refroidissement, la plaque est immergée dans l'eau où l'on observe le décollement du film.

Après évaporation du solvant, le film obtenu présente une épaisseur moyenne de 20 µm.
Un échantillon de ce film a ensuite été testé dans une cellule de perméation circulaire d'un diamètre efficace de 5,5 cm placé dans une enceinte thermostatée.

La face amont de la membrane ainsi testée a été balayée pendant 20 jours avec un flux gazeux de 10 Nl/h composé de normal butane et d'isobutane ou de normal butène et d'isobutène alors que le compartiment en aval de la membrane, dans lequel est collecté le perméat, est balayé par un flux d'azote de 1 Nl/h à la pression atmosphérique.
La composition des différents fluides entrant et sortant des différents compartiments de la cellule de perméation est obtenue par chromatographie en phase gazeuse.
Les performances en régime permanent du film, restées constantes pendant 20 jours, sont les suivantes :

**Tableau 3. Performances du polyimide 6FDA-BDAF (Pression charge = 0,15 MPa)**

| Composition de la charge | Température du film | Perméabilité normal butane (ou normal butène) | Sélectivité en mélange |
|---|---|---|---|
| normal butane / isobutane (50/50) | 150°C | 11 barrer | 8 |
| normal butène / isobutène (50/50) | 150°C | 14 barrer | 7 |

### Exemple 2 (selon l'invention)

La synthèse du film polyimide selon l'exemple 2 est réalisée en deux étapes. Lors de la première étape, le dianhydride de type 6FDA est mis en contact avec la diamine de type BDAF dans le solvant N-méthylpyrrolidone (NMP) sous atmosphère inerte et en milieu anhydre.
Après 3 heures d'agitation à température ambiante, le polyamide acide est obtenu. La seconde étape consiste en une déshydratation thermique de ce polyamide acide par chauffage (30 min à 100 °C, 1 heure à 160 °C, 1 heure à 180 °C et deux heures à 200 °C).
Le polyimide ainsi obtenu est alors précipité dans de l'eau, broyé, et séché comme décrit dans l'exemple 1 de la présente invention. La viscosité inhérente du polymère ainsi obtenu est de 0,55 dl/g.
Le polymère est ensuite redissout dans le solvant NMP à une concentration de 10 % massique. La solution limpide est ensuite filtrée sous une pression de 0,2 MPa sur un filtre de type Millipore ayant un seuil de coupure de 1 µm.

Cette solution est ensuite mise sous forme d'un film à l'aide d'une barre spiralée de 300 µm sur une plaque de verre préalablement dégraissée à l'acétone puis séchée.
La plaque est introduite dans une étuve. L'évaporation du solvant est effectuée par une élévation progressive de la température jusqu'à 200°C.

Après évaporation du solvant, le film obtenu présente une épaisseur moyenne de 32 µm.
Les performances du film selon l'exemple 2 ont été obtenues dans des conditions de tests identiques à celles décrites dans l'exemple 1.

**Tableau 4. Performances du polyimide 6FDA-BDAF (Pression charge =0,15 MPa)**

| Composition de la charge | Température du film | Perméabilité normal butane (ou normal butène) | Sélectivité en mélange |
|---|---|---|---|
| normal butane / isobutane (50/50) | 150°C | 0,4 barrer | 8 |
| normal butène / isobutène (50/50) | 150°C | 0,6 barrer | 7 |

### Exemple 3 (selon l'invention)

Le polymère BTDA-BDAF faisant l'objet de l'exemple 3 est le résultat de la polycondensation du dianhydride de l'acide 3,3',4,4'-benzophénonetétracarboxylique (BTDA) et de la diamine 9,9'bis-(4-aminophényl)-fluorène (BDAF) en mélange équimolaire.
Après purification des monomères par recristallisation dans des solvants appropriés, la polycondensation du polyimide BTDA-BDAF est réalisée en deux étapes: dans un premier temps, le polyamide acide est réalisé, puis le polyimide est obtenu dans une deuxième étape de cyclisation par voie chimique.
Lors de la première étape de polymérisation, le mélange de la dianhydride et de la diamine est effectué sous atmosphère inerte et en milieu anhydre dans le solvant NMP.
L'étape de cyclo-déshydratation est effectuée par ajout goutte à goutte d'un mélange cyclisant composé de triéthylamine et d'anhydride acétique en mélange dans le solvant de synthèse.
Le polyimide ainsi obtenu est alors précipité dans de l'eau puis broyé. Il est ensuite filtré, rincé, puis séché à l'étuve sous vide en augmentant progressivement la température jusqu'à atteindre 150 °C. La viscosité inhérente du polymère ainsi obtenu était de 0,8 dl/g dans la NMP.

Le matériau sous forme de broyé est ensuite mis en solution dans de la NMP à une concentration massique de 10 % sous l'effet d'une bonne agitation mécanique à température ambiante. La solution limpide est ensuite filtrée sous une pression de 0,2 MPa sur un filtre de type Millipore ayant un seuil de coupure de 1 µm.
Cette solution est ensuite mise sous forme d'un film à l'aide d'une barre spiralée de 300 µm sur une plaque de verre préalablement dégraissée à l'acétone puis séchée.
La plaque est introduite dans une étuve. L'évaporation du solvant est effectuée par une élévation progressive de la température jusqu'à 200°C. La température finale est maintenue en palier pendant deux heures. Après refroidissement, la plaque est immergée dans l'eau où l'on observe le décollement du film.

Après évaporation du solvant, le film obtenu présentait une épaisseur moyenne de 24 µm. Les performances du film selon l'exemple 4 ont été obtenues dans des conditions de tests identiques à celles décrites dans l'exemple 1.

**Tableau 4. Performances du film polyimide BTDA-BDAF (Pression charge = 0,15 MPa)**

| Composition de la charge | Température du film | Perméabilité normal butane (ou normal butène) | Sélectivité en mélange |
|---|---|---|---|
| normal butane / isobutane (50/50) | 150°C | 2 barrer | 8 |
| normal butène / isobutène (50/50) | 150°C | 2,5 barrer | 8 |

### Exemple 4 (selon l'invention)

Le polymère BPDA-BDAF faisant l'objet de l'exemple 4 est le résultat de la polycondensation du dianhydride de l'acide 3,3',4,4'-biphényltétracarboxylique (BPDA) et de la diamine 9,9'bis-(4-aminophényl)-fluorène (BDAF) en mélange équimolaire.

Les monomères sont introduits dans le solvant DMAC sous atmosphère inerte et en milieu anhydre.
Après 8 heures d'agitation à température ambiante, le polyamide acide est obtenu. La seconde étape consiste en une déshydratation thermique de ce polyamide acide par chauffage (une heure à 100 °C, trois heures à 200 °C).
Après évaporation du solvant, le film obtenu présente une épaisseur moyenne de 40 µm.
Les performances du film tel qu'obtenu selon l'exemple 4 ont été obtenues dans des conditions de tests identiques à celles décrites dans l'exemple 1.

**Tableau 6. Performances du polyimide BTDA-BDAF (Pression charge = 0,9 MPa)**

| Composition de la charge | Température du film | Perméabilité normal butane (ou normal butène) | Sélectivité en mélange |
|---|---|---|---|
| normal butane / isobutane (50/50) | 150°C | 0,2 barrer | 10 |
| normal butène / isobutène (50/50) | 150°C | 0,23 barrer | 8 |

### Exemple 5 (exemple selon l'invention)

Une membrane composite offrant une couche sélective selon le mode décrit dans la présente invention a été synthétisée en enduisant des fibres creuses en polyoxyde de phénylène produite par la société Parker Filtration (Parker Hannifin SA, UCC France, Rue Albert Calette, BP6, 41260 La Chaussée St Victor, France), selon le mode suivant :
Le polymère de type 6FDA-BDAF obtenu selon la méthode décrite dans l'exemple 2 est mis en solution dans de la NMP à une concentration massique de 5% sous l'effet d'une bonne agitation mécanique à température ambiante.
La solution limpide est ensuite filtrée sous une pression de 0,2 MPa sur un filtre de type Millipore ayant un seuil de coupure de 1 µm. On trempe la fibre en poly-2,6- diméthyl-1,4-phénylène oxyde dans la solution de polymère dilué puis on l'extrait verticalement de la solution en prenant soin que l'excès de solution soit évacuée par gravité.
La fibre enduite est ensuite mise à sécher verticalement dans une étude sous atmosphère inerte selon la progression thermique suivante : 30 min à 100 °C, 2 heure à 160 °C.
Des échantillons de fibres enduites analysées par microscopie électronique à balayage ont montré que la couche de polyimide sélectif aux oléfines présentait une épaisseur comprise entre 0,1 et 0,5 µm.
Un faisceau de fibres a ensuite été serti dans une calandre avec de la résine époxy et a été soumis à des tests de séparation de mélange normal butane / isobutane à l'état gazeux dans les conditions décrites dans l'exemple 1.
Lors de tests de séparation de mélanges constitués de normal butane et d'isobutane à des ratios molaires respectifs de 50% et 50%, à une température de 150°C et des pressions en amont et en aval de la membrane respectivement de 0,15 et 0,1 MPa, la sélectivité en mélange des fibres composites étaient de 8.

## Revendications

1. Procédé de séparation par membrane permettant l'extraction sélective d'un hydrocarbure linéaire contenu dans un mélange composé au moins dudit hydrocarbure linéaire et d'un hydrocarbure branché, la couche sélective de la ladite membrane étant constituée d'un film dense polymère dont la structure chimique contient un groupement bis-phényl-9,9-fluorène.

2. Procédé de séparation par membrane selon la revendication 1 dans lequel la couche sélective de la membrane polymère contenant le groupement bis-phényl-9,9-fluorène est sélectionnée dans le groupe constitué par les polymères des familles suivantes: les polyimides, les polyamides, les polycarbonates, les polysulfones, les poly(amides imides), les poly(éther sulfones), les polyesters, ou par les copolymères ou mélanges de polymères de ces familles.

3. Procédé de séparation par membrane selon la revendication 1 dans lequel la couche sélective de la membrane polymère contenant le groupement bis-phényl-9,9-fluorène appartient à la famille des polyimides.

4. Procédé de séparation par membrane selon la revendication 1 dans lequel la couche sélective de la membrane polymère contenant le groupement bis-phényl-9,9-fluorène appartient à la famille des polyamides.

5. Procédé de séparation par membrane selon la revendication 1 dans lequel la couche sélective de la membrane polymère contenant le groupement bis-phényl-9,9-fluorène appartient à la famille des polycarbonates.

6. Procédé de séparation par membrane selon la revendication 1 dans lequel la couche sélective de la membrane polymère contenant un groupement bis-phényl-9,9-fluorène appartient à la famille des polysulfones

7. Procédé de séparation par membrane selon la revendication 3, dans lequel le polymère constitutif de la couche sélective de la membrane est un polyimide ayant été synthétisé avec le dianhydride de l'acide 2,2-bis(3,4-dicarboxyphényl)hexafluoropropane.

8. Procédé de séparation par membrane selon la revendication 3, dans lequel le polymère constitutif de la couche sélective de la membrane est un polyimide ayant été synthétisé avec le dianhydride de l'acide 3,3',4,4'-biphényltétracarboxylique.

9. Procédé de séparation par membrane selon la revendication 3, dans lequel le polymère constitutif de la couche sélective de la membrane est un polyimide ayant été synthétisé avec le dianhydride de l'acide 3,3',4,4'-benzophénonetétracarboxylique

10. Procédé de séparation par membrane selon la revendication 3, dans lequel le polymère constitutif de la couche sélective de la membrane est un polyimide ayant été synthétisé avec la diamine 1,3-diamino-2,4,6-triméthylbenzène.

11. Procédé de séparation par membrane selon la revendication 3, dans lequel le polymère constitutif de la couche sélective de la membrane est un polyimide issu de la polycondensation du dianhydride de l'acide 2,2-bis(3,4-dicarboxyphényl)hexafluoropropane et de la diamine 9,9-bis(4-aminophényl)fluorène.

12. Procédé de séparation par membrane selon la revendication 3, dans lequel le polymère constitutif de la couche sélective de la membrane est un polyimide issu de la polycondensation du dianhydride de l'acide 3,3',4,4'-biphényltétracarboxylique et de la diamine 9,9-bis(4-aminophényl)fluorène.

13. Procédé de séparation par membrane selon la revendication 3, dans lequel le polymère constitutif de la couche sélective de la membrane est un polyimide issu de la polycondensation du dianhydride de l'acide 3,3',4,4'-benzophénonetétracarboxylique et de la diamine 9,9-bis(4-aminophényl)fluorène.

14. Procédé de séparation par membrane selon l'une quelconque des revendications 1 à 13 dans lequel l'hydrocarbure linéaire à séparer contient 4 atomes de carbone.

15. Procédé de séparation par membrane selon l'une quelconque des revendications 1 à 13 dans lequel l'hydrocarbure linéaire à séparer contient 5 atomes de carbone.

16. Procédé de séparation par membrane selon l'une quelconque des revendications 1 à 13 dans lequel l'hydrocarbure linéaire à séparer contient 6 atomes de carbone.

17. Procédé de séparation par membrane selon l'une quelconque des revendications 1 à 16 dans lequel la membrane sélective est déposée à la surface d'un support de type fibre creuse à base de poly-2,6- diméthyl-1,4-phénylène oxyde.

18. Procédé de séparation par membrane selon l'une quelconque des revendications 1 à 17 dans lequel la température dudit procédé est comprise entre 40 °C et 200 °C et la pression du mélange à séparer est comprise entre 1 et 40 bar.

19. Procédé de séparation par membrane selon l'une quelconque des revendications 1 à 17 dans lequel la température dudit procédé est comprise entre 50 °C et 150 °C et la pression du mélange à séparer est comprise entre 1 et 20 bar.

## Claims

1. Process for membrane separation that allows the selective extraction of a linear hydrocarbon that is contained in a mixture that consists of at least said linear hydrocarbon and a branched hydrocarbon, whereby the selective layer of said membrane consists of a dense polymer film whose chemical structure contains a bisphenyl-9,9-fluorene group.

2. Process for membrane separation according to claim 1, in which the selective layer of the polymer membrane that contains the bis-phenyl-9,9-fluorene group is selected from the group that consists of the polymers of the following families: the polyimides, the polyamides, the polycarbonates, the polysulfones, the poly(amide imides), the poly(ether sulfones), the polyesters, or the copolymers or polymer mixtures of these families.

3. Process for membrane separation according to claim 1, in which the selective layer of the polymer membrane that contains the bis-phenyl-9,9-fluorene group belongs to the family of polyimides.

4. Process for membrane separation according to claim 1, in which the selective layer of the polymer membrane that contains the bis-phenyl-9,9-fluorene group belongs to the family of polyamides.

5. Process for membrane separation according to claim 1, in which the selective layer of the polymer membrane that contains the bis-phenyl-9,9-fluorene group belongs to the family of polycarbonates.

6. Process for membrane separation according to claim 1, in which the selective layer of the polymer membrane that contains a bis-phenyl-9,9-fluorene group belongs to the family of polysulfones.

7. Process for membrane separation according to claim 3, in which the polymer that constitutes the selective layer of the membrane is a polyimide that has been synthesized with the 2,2-bis(3,4-dicarboxyphenyl)hexafluoropropanoic acid dianhydride.

8. Process for membrane separation according to claim 3, in which the polymer that constitutes the selective layer of the membrane is a polyimide that has been synthesized with the 3,3',4,4'-biphenyltetracarboxylic acid dianhydride.

9. Process for membrane separation according to claim 3, in which the polymer that constitutes the selective layer of the membrane is a polyimide that has been synthesized with the 3,3',4,4'-benzophenonetetracarboxylic acid dianhydride.

10. Process for membrane separation according to claim 3, in which the polymer that constitutes the selective layer of the membrane is a polyimide that has been synthesized with the 1,3-diamino-2,4,6-trimethylbenzene diamine.

11. Process for membrane separation according to claim 3, in which the polymer that constitutes the selective layer of the membrane is a polyimide that is obtained from the polycondensation of the 2,2-bis(3,4-dicarboxyphenyl)hexa-fluoropropanoic acid dianhydride and the 9,9-bis(4-aminophenyl)fluorene diamine.

12. Process for membrane separation according to claim 3, in which the polymer that constitutes the selective layer of the membrane is a polyimide that is obtained from the polycondensation of the 3,3',4,4'-biphenyltetracarboxylic acid dianhydride and the 9,9-bis(4-aminophenyl)fluorene diamine.

13. Process for membrane separation according to claim 3, in which the polymer that constitutes the selective layer of the membrane is a polyimide that is obtained from the polycondensation of the 3,3',4,4'-benzophenone-tetracarboxylic acid dianhydride and the 9,9-bis(4-aminophenyl)fluorene diamine.

14. Process for membrane separation according to any of claims 1 to 13, in which the linear hydrocarbon that is to be separated contains 4 carbon atoms.

15. Process for membrane separation according to any of claims 1 to 13, in which the linear hydrocarbon that is to be separated contains 5 carbon atoms.

16. Process for membrane separation according to any of claims 1 to 13, in which the linear hydrocarbon that is to be separated contains 6 carbon atoms.

17. Process for membrane separation according to any of claims 1 to 16, in which the selective membrane is deposited on the surface of a hollow-fiber-type substrate that is based on poly-2,6-dimethyl-1,4-phenylene oxide.

18. Process for membrane separation according to any of claims 1 to 17, in which the temperature of said process is encompassed between 40°C and 200°C, and the pressure of the mixture that is to be separated is between 1 and 40 bar.

19. Process for membrane separation according to any of claims 1 to 17, in which the temperature of said process is between 50°C and 150°C, and the pressure of the mixture that is to be separated is between 1 and 20 bar.

## Patentansprüche

1. Membrantrennverfahren, das die selektive Extraktion eines linearen Kohlenwasserstoffs ermöglicht, der in einem Gemisch enthalten ist, das aus mindestens dem linearen Kohlenwasserstoff und einem verzweigten Kohlenwasserstoff zusammengesetzt ist, wobei die selektive Schicht der Membran aus einem dichten Polymerfilm besteht, dessen chemische Struktur eine Bisphenyl-9,9-fluoren-Gruppe enthält.

2. Membrantrennverfahren nach Anspruch 1, wobei die selektive Schicht der Polymermembran, die die Bisphenyl-9,9-fluoren-Gruppe enthält, ausgewählt ist aus der Gruppe bestehend aus den Polymeren der folgenden Familien: den Polyimiden, den Polyamiden, den Polycarbonaten, den Polysulfonen, den Poly(amidimiden), den Poly(ethersulfonen), den Polyestern oder den Copolymeren oder Polymergemischen dieser Familien .

3. Membrantrennverfahren nach Anspruch 1, wobei die selektive Schicht der Polymermembran, die die Bisphenyl-9,9-fluoren-Gruppe enthält, zur Familie der Polyimide gehört.

4. Membrantrennverfahren nach Anspruch 1, wobei die selektive Schicht der Polymermembran, die die Bisphenyl-9,9-fluoren-Gruppe enthält, zur Familie der Polyamide gehört.

5. Membrantrennverfahren nach Anspruch 1, wobei die selektive Schicht der Polymermembran, die die Bisphenyl-9,9-fluoren-Gruppe enthält, zur Familie der Polycarbonate gehört.

6. Membrantrennverfahren nach Anspruch 1, wobei die selektive Schicht der Polymermembran, die eine Bisphenyl-9,9-fluoren-Gruppe enthält, zur Familie der Polysulfone gehört.

7. Membrantrennverfahren nach Anspruch 3, wobei das Polymer, das die selektive Schicht der Membran bildet, ein Polyimid ist, das mit 2,2-Bis(3,4-dicarboxyphenyl)hexafluorpropansäuredianhydrid synthetisiert wurde.

8. Membrantrennverfahren nach Anspruch 3, wobei das Polymer, das die selektive Schicht der Membran bildet, ein Polyimid ist, das mit 3,3',4,4'-Biphenyltetracarbonsäuredianhydrid synthetisiert wurde.

9. Membrantrennverfahren nach Anspruch 3, wobei das Polymer, das die selektive Schicht der Membran bildet, ein Polyimid ist, das mit 3,3',4,4'-Benzophenontetracarbonsäuredianhydrid synthetisiert wurde.

10. Membrantrennverfahren nach Anspruch 3, wobei das Polymer, das die selektive Schicht der Membran bildet, ein Polyimid ist, das mit 1,3-Diamino-2,4,6-trimethylbenzendiamin synthetisiert wurde.

11. Membrantrennverfahren nach Anspruch 3, wobei das Polymer, das die selektive Schicht der Membran bildet, ein Polyimid ist, das aus der Polykondensation von 2,2-Bis(3,4-dicarboxyphenyl)hexafluorpropansäuredianhydrid und 9,9-Bis(4-aminophenyl)fluorendiamin stammt.

12. Membrantrennverfahren nach Anspruch 3, wobei das Polymer, das die selektive Schicht der Membran bildet, ein Polyimid ist, das aus der Polykondensation von 3,3',4,4'-Biphenyltetracarbonsäuredianhydrid und 9,9-Bis(4-aminophenyl)fluorendiamin stammt.

13. Membrantrennverfahren nach Anspruch 3, wobei das Polymer, das die selektive Schicht der Membran bildet, ein Polyimid ist, das aus der Polykondensation von 3,3',4,4'-Benzophenontetracarbonsäuredianhydrid und 9,9-Bis(4-aminophenyl)fluorendiamin stammt.

14. Membrantrennverfahren nach irgendeinem der Ansprüche 1 bis 13, wobei der zu trennende lineare Kohlenwasserstoff 4 Kohlenstoffatome enthält.

15. Membrantrennverfahren nach irgendeinem der Ansprüche 1 bis 13, wobei der zu trennende lineare Kohlenwasserstoff 5 Kohlenstoffatome enthält.

16. Membrantrennverfahren nach irgendeinem der Ansprüche 1 bis 13, wobei der zu trennende lineare Kohlenwasserstoff 6 Kohlenstoffatome enthält.

17. Membrantrennverfahren nach irgendeinem der Ansprüche 1 bis 16, wobei die selektive Membran auf der Oberfläche eines Trägers vom Hohlfasertyp auf der Basis von Poly-2,6-dimethyl-1,4-phenylenoxid abgeschieden ist.

18. Membrantrennverfahren nach irgendeinem der Ansprüche 1 bis 17, wobei die Temperatur des Verfahrens im Bereich zwischen 40 °C und 200 °C liegt und der Druck des zu trennenden Gemischs im Bereich zwischen 1 und 40 Bar liegt.

19. Membrantrennverfahren nach irgendeinem der Ansprüche 1 bis 17, wobei die Temperatur des Verfahrens im Bereich zwischen 50 °C und 150 °C liegt und der Druck des zu trennenden Gemischs im Bereich zwischen 1 und 20 Bar liegt.
